# EUROPÄISCHE PATENTSCHRIFT

(11) **EP 4 322 912 B1**
(45) Veröffentlichungstag und Bekanntmachung des Hinweises auf die Patenterteilung: **07.05.2025**
(21) Anmeldenummer: 22713901.1
(22) Anmeldetag: 10.03.2022
(51) Int. Cl.: A61K 8/37, A61K 8/9789, A61Q 19/00

(54) **CREME-BILDENDES HAUTPFLEGEÖL**
CREAM-FORMING SKIN CARE OIL
HUILE DE SOIN DE LA PEAU FORMANT UNE CRÈME

(30) Priorität: 12.04.2021 DE 102021203556
(43) Veröffentlichungstag der Anmeldung: 21.02.2024
(73) Patentinhaber: Beiersdorf AG, 22529 Hamburg (DE)
(72) Erfinder: VOIGT, Nadine, 22111 Hamburg (DE); JAPP, Christina, 21149 Hamburg (DE)
(74) Vertreter: Beiersdorf AG
(86) Internationale Anmeldenummer: PCT/EP2022/056137
(87) Internationale Veröffentlichungsnummer: WO 2022/218615

(56) Entgegenhaltungen:
- EP-A2- 2 749 651
- DATABASE GNPD [online] MINTEL; 8 April 2020 (2020-04-08), ANONYMOUS: "Jacqueline's Blend Extraordinary Face Oil", XP055936868, retrieved from https://www.gnpd.com/sinatra/recordpage/7529127/ Database accession no. 7529127

## Beschreibung

Die vorliegende Erfindung betrifft eine wasserfreie kosmetische Zubereitung enthaltend Diisostearoyl Polyglyceryl-3 Dimer Dilinoleate, Polyglyceryl-4 Diisostearate, fermentiertes Olivenöl und ein oder mehrere Öle mit einer Oberflächenspannung von weniger als 31 mN/m.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Hautpflegeprodukte, in der Regel Crémes, Salben oder Lotionen, dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte werde in der Regel in zwei unterschiedlichen Formen angeboten. Der überwiegende Teil der Produkte basiert auf Emulsionen, bei denen eine Wasser- und eine Ölphase optisch homogen miteinander vermengt vorliegen. Ein kleinerer Teil der Produkte wird in Form von Hautölen ohne eigene Wasserphase dargereicht.

Nicht zuletzt aus Gründen der Volumen- und Gewichtsersparnis und der daraus resultierenden Schonung natürlicher Ressourcen bei Herstellung, Transport und Lagerung sind in jüngerer Zeit die Hautpflegeöle wieder stärker in den Fokus der Produktentwicklung kosmetischer Zubereitungen geraten. Wenn bei der Pflege der Haut die Rückfettung mit Lipid-Komponenten im Vordergrund steht, ist diese Produktform von besonderem Interesse.

Nachteilig am Stande der Technik ist jedoch der Umstand, dass sich die Hautpflegeöle nur schlecht auf nasser oder feuchter Haut auftragen lassen. Meist perlen sie auf der Haut ab, ohne dass die Lipid-Komponenten von der Haut aufgenommen werden können.

Mintel GNPD Record ID 7529127 "Jacqueline's Blend Extraordinary Face Oil", Apr 2020, zeigt eine wasserfreie Pflegeölzusammensetzung enthaltend fermentiertes Olivenöl und verschiedene Öle. EP2749651 A2 offenbart ein fermentiertes Pflanzenöl, z.B. fermentiertes Olivenöl, dass verbesserte emulgierende Eigenschaften hat.

Es war daher die Aufgabe der vorliegenden Erfindung, ein Hautpflegeöl zu entwickeln, welches sich gut auf nasser Haut auftragen und verteilen lässt. Idealerweise sollte das Hautpflegeöl besonders haut- und umweltfreundlich sein und weitgehend auf Rohstoffen natürlichen Ursprungs basieren.

Überraschend gelöst wird die Aufgabe durch eine wasserfreie kosmetische Zubereitung enthaltend
a) Diisostearoyl Polyglyceryl-3 Dimer Dilinoleate
b) Polyglyceryl-4 Diisostearate,
c) fermentiertes Olivenöl
d) ein oder mehrere Öle mit einer Oberflächenspannung von weniger als 31 mN/m.

Dabei wird die Oberflächenspannung erfindungsgemäß mit dem Tensiometer K100 von der Firma Krüss (Du Noüy-Ring) mit einem Ring gegen Luft bei 20°C, gemessen.

Zwar gab es in der Vergangenheit wiederholt Versuche, Mischungen von Lipiden und Emulgatoren als Hautpflegeöle anzubieten, doch hatten diese Ansätze in der Regel den Nachteil, dass sich bei der Verwendung von O/W-Emulgatoren sich diese nicht in ausreichender Menge in die Ölphase einarbeiten ließen und beim Einsatz von W/O-Emulgatoren die Emulsionsbildung beim Verreiben auf nasser Haut zu wünschen übrig ließ. Darüber hinaus sind die Systeme des Standes der Technik meist nicht besonders haut- und umweltfreundlich, da sie nicht auf der Basis natürlicher Rohstoffe hergestellt wurden.

Hingegen war es für den Fachmann überraschend und nicht vorhersehbar, dass sich beim Verreiben der erfindungsgemäßen Zubereitung auf der feuchten Haut eine sichtbare weiße Pflegeemulsion bildet.

Erfindungsgemäß werden unter "wasserfrei" Zubereitungen verstanden, bei denen außer den in den Rohstoffen üblicherweise enthaltenden Wasserspuren kein Wasser enthalten oder zugesetzt wird.

Es ist daher erfindungsgemäß bevorzugt, wenn die Zubereitung weniger als 1 Gewichts-%, bezogen auf das Gesamtgewicht der Zubereitung, an Wasser enthält.

Es ist vorteilhaft im Sinne der vorliegenden Erfindung, wenn Diisostearoyl Polyglyceryl-3 Dimer Dilinoleate in einer Konzentration von 0,5 bis 4 Gewichts-%, bezogen auf das Gesamtgewicht der Zubereitung eingesetzt wird. Dabei ist der Konzentrationsbereich von 2 bis 3 Gewichts-%, bezogen auf das Gesamtgewicht der Zubereitung, erfindungsgemäß bevorzugt.

Auch ist es erfindungsgemäß von Vorteil, wenn Polyglyceryl-4 Diisostearate in einer Konzentration von 0,5 bis 4 Gewichts-%, bezogen auf das Gesamtgewicht der Zubereitung eingesetzt wird. Dabei ist der Konzentrationsbereich von 2 bis 3 Gewichts-%, bezogen auf das Gesamtgewicht der Zubereitung, erfindungsgemäß bevorzugt.

Erfindungsgemäß vorteilhafte Ausführungsformen der vorliegenden Erfindung sind dadurch gekennzeichnet, dass die Zubereitung ein oder mehrere Fettalkohole enthält.

Dabei ist es erfindungsgemäß bevorzugt, einen oder mehrere Fettalkohole gewählt aus der Liste der folgenden Verbindungen einzusetzen: Cetearyl Alcohol, Myristyl Alcohol, Stearyl Alcohol Es ist dabei erfindungsgemäß vorteilhaft, wenn die Gesamtmenge an Fettalkoholen 1 bis 4 Gewichts.-%, bezogen auf das Gesamtgewicht der Zubereitung beträgt.

Ferner ist es erfindungsgemäß von Vorteil, wenn die Zubereitung Kakaobutter enthält. In einem solchen Falle ist es erfindungsgemäß bevorzugt, wenn die Kakaobutter in einer Menge von 1 bis 4 Gewichts-%, bezogen auf das Gesamtgewicht der Zubereitung eingesetzt wird.

Es ist vorteilhaft im Sinne der vorliegenden Erfindung, wenn als Öle mit einer Oberflächenspannung von weniger als 31 mN/m, ein oder mehrere Verbindungen gewählt aus der Gruppe Octyldodecanol, Capryl/Caprinsäure Triglycerid (INCI Caprylic/Capric Triglyceride), Isopropylpalmitat (INCI: Isopropyl Palmitate), Sheabuterethylester (INCI: Shea Butter Ethyl Esters), Rübsenöl (INCI: Brassica Campestris Seed Oil) eingesetzt werden.

Es ist erfindungsgemäß bevorzugt, wenn das Olivenöl durch Pseudozyma sp.SY-16[KCTC 8950P] fermentiert wurde.

Erfindungsgemäß besonders bevorzugt ist es dabei, wenn als fermentiertes Olivenöl die Substanz mit der INCI Pseudozyme Epicola/Olive Fruit Oil eingesetzt wird. Dieses kann beispielsweise unter dem Handelsnamen "Fermentoil Olive" bei der Firma LABIO Co., Ltd. erworben werden.

Die erfindungsgemäß vorteilhaften Ausführungsformen sind dabei dadurch gekennzeichnet, dass fermentiertes Olivenöl in einer Konzentration von 0,25 bis 2 Gewichts-%, bezogen auf das Gesamtgewicht der Zubereitung eingesetzt wird. Dabei ist der Konzentrationsbereich von 0,5 bis 1 Gewichts-%, bezogen auf das Gesamtgewicht der Zubereitung, erfindungsgemäß bevorzugt.

Es ist erfindungsgemäß von Vorteil, wenn die Gesamtmenge an Ölen mit einer Oberflächenspannung von weniger als 31 mN/m, mindestens 60 Gewichts-%, bezogen auf das Gesamtgewicht der Zubereitung beträgt. Erfindungsgemäß bevorzugt, wird die Differenz zum Endprodukt durch Auffüllen mit diesen Ölen ausgeglichen (zu ad. 100 Gewichts-%).

Erfindungsgemäß vorteilhafte Ausführungsformen der vorliegenden Erfindung sind ferner dadurch gekennzeichnet, dass die Zubereitung einen oder mehrere Wirkstoffe gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; Thiamidol; 8-Hexadecen-1,16-dicarbonsäure, Glycerylglycose, (2-Hydroxyethyl)harnstoff, Vitamin E bzw. seine Derivate, Hyaluronsäure und/oder deren Salze, Lecithin und/oder Licochalcon A, enthält.

Auch ist es erfindungsgemäß von Vorteil, wenn die Zubereitung 2,6-Di-*tert*-butyl-*p*-kresol (INCI: BHT) oder Ascorbyl Palmitate enthält.

Nicht zuletzt sind die erfindungsgemäß vorteilhaften Ausführungsformen dadurch gekennzeichnet, dass die Zubereitung frei ist von, Phenoxyethanol, 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen, Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | Beispielrezeptur 1 | Beispielrezeptur 2 | Beispielrezeptur 3 | Beispielrezeptur 4 |
|---|---|---|---|---|
| INCI | m [%] | m [%] | m [%] | m [%] |
| Isopropyl Palmitate | | | | 87.00 |
| Caprylic/Capric Triglyceride | 47.00 | 59.00 | | |
| Octyldodecanol | 29.00 | | 2.00 | 2.00 |
| Myristyl Alcohol | 2.00 | 2.00 | 2.00 | 2.00 |
| Diisostearoyl Polyglyceryl-3 Dimer Dilinoleate | 2.00 | 3.00 | 3.00 | 3.00 |
| Theobroma Cacao Seed Butter | 2.00 | 2.00 | 2.00 | 2.00 |
| Polyglyceryl-4 Diisostearate/Polyhydroxystearate/Sebacate | 2.00 | 3.00 | 3.00 | 3.00 |
| Brassica Campestris Seed Oil | 15.00 | | | |
| Octyldodecanol | | 30.00 | | |
| Shea Butter Ethyl Esters + Citric Acid | | | 87.00 | |
| Pseudozyma Epicola/Olive Fruit Oil Ferment Filtrate | 1.00 | 1.00 | 1.00 | 1.00 |
| | | | | |
| Summen: | 100.00 | 100.00 | 100.00 | 100.00 |

### Vergleichsrezepturen:

Die Beispielrezeptur 5 erzeugt durch die besondere Zusammensetzung beim Auftragen auf feuchter Haut einen weißen Pflegefilm, der sich leicht verteilen lässt und gut absorbiert. Die dazu im Vergleich aufgeführte Beispielrezeptur 6 zeigt eine deutlich schlechtere Performance. Diese Zubereitung ohne fermentiertes Olivenöl, mit dem Hauptbestandteil Helianthus Annuus Seed Oil (Oberflächenspannung > 31 mN/m) wird beim Auftragen auf feuchter Haut kaum bis gar nicht weiß und zeigt eine schlechtere Verteilbarkeit und Absorption.

| | Beispielrezeptur 5 | Beispielrezeptur 6 |
|---|---|---|
| INCI | m [%] | m [%] |
| Caprylic/Capric Triglyceride | 34.00 | |
| Octyldodecanol | | 10.00 |
| Myristyl Alcohol | 2.00 | 2.00 |
| Diisostearoyl Polyglyceryl-3 Dimer Dilinoleate | 3.00 | 3.00 |
| Theobroma Cacao Seed Butter | 2.00 | 2.00 |
| Helianthus Annuus Seed Oil | | 80.00 |
| Polyglyceryl-4 Diisostearate/Polyhydroxystearate/Sebacate | 3.00 | 3.00 |
| Octyldodecanol | 30.00 | |
| Shea Butter Ethyl Esters + Citric Acid | 25.00 | |
| Pseudozyma Epicola/Olive Fruit Oil Ferment Filtrate | 1.00 | |
| Summen: | 100.00 | 100.00 |

## Patentansprüche

1. Wasserfreie kosmetische Zubereitung enthaltend
a) Diisostearoyl Polyglyceryl-3 Dimer Dilinoleate
b) Polyglyceryl-4 Diisostearate,
c) fermentiertes Olivenöl
d) ein oder mehrere Öle mit einer Oberflächenspannung von weniger als 31 mN/m.

2. Kosmetische Zubereitung nach Anspruch 1, **dadurch gekennzeichnet, dass** die Zubereitung weniger als 1 Gewichts-%, bezogen auf das Gesamtgewicht der Zubereitung, an Wasser enthält.

3. Kosmetische Zubereitung nach einem der vorhergehenden Ansprüche, **dadurch gekennzeichnet, dass** die Zubereitung ein oder mehrere Fettalkohole enthält.

4. Kosmetische Zubereitung nach einem der vorhergehenden Ansprüche, **dadurch gekennzeichnet, dass** die Zubereitung Kakaobutter enthält.

5. Kosmetische Zubereitung nach einem der vorhergehenden Ansprüche, **dadurch gekennzeichnet, dass** als Öle mit einer Oberflächenspannung von weniger als 31 mN/m, ein oder mehrere Verbindungen gewählt aus der Gruppe Octyldodecanol, Capryl/Caprinsäure Triglycerid (INCI Caprylic/Capric Triglyceride), Isopropylpalmitat (INCI: Isopropyl Palmitate), Sheabuterethylester (INCI: Shea Butter Ethyl Esters), Rübsenöl (INCI: Brassica Campestris Seed Oil) eingesetzt werden.

6. Kosmetische Zubereitung nach einem der vorhergehenden Ansprüche, **dadurch gekennzeichnet, dass** das Olivenöl durch Pseudozyma sp.SY-16[KCTC 8950P] fermentiert wurde.

7. Kosmetische Zubereitung nach einem der vorhergehenden Ansprüche, **dadurch gekennzeichnet, dass** als fermentiertes Olivenöl die Substanz mit der INCI Pseudozyme Epicola/Olive Fruit Oil eingesetzt wird.

8. Kosmetische Zubereitung nach einem der vorhergehenden Ansprüche, **dadurch gekennzeichnet, dass** Diisostearoyl Polyglyceryl-3 Dimer Dilinoleate in einer Konzentration von 2 bis 4 Gewichts-%, bezogen auf das Gesamtgewicht der Zubereitung eingesetzt wird.

9. Kosmetische Zubereitung nach einem der vorhergehenden Ansprüche, **dadurch gekennzeichnet, dass** Polyglyceryl-4 Diisostearate in einer Konzentration von 2 bis 4 Gewichts-%, bezogen auf das Gesamtgewicht der Zubereitung eingesetzt wird.

10. Kosmetische Zubereitung nach einem der vorhergehenden Ansprüche, **dadurch gekennzeichnet, dass** fermentiertes Olivenöl in einer Konzentration von 0,5 bis 2 Gewichts-%, bezogen auf das Gesamtgewicht der Zubereitung eingesetzt wird.

11. Kosmetische Zubereitung nach einem der vorhergehenden Ansprüche, **dadurch gekennzeichnet, dass** die Gesamtmenge an Ölen mit einer Oberflächenspannung von weniger als 31 mN/m, mindestens 60 Gewichts-%, bezogen auf das Gesamtgewicht der Zubereitung beträgt.

12. Kosmetische Zubereitung nach einem der vorhergehenden Ansprüche, **dadurch gekennzeichnet, dass** die Gesamtmenge an Fettalkoholen 2 bis 4 Gewichts.-%, bezogen auf das Gesamtgewicht der Zubereitung beträgt.

13. Kosmetische Zubereitung nach einem der vorhergehenden Ansprüche, **dadurch gekennzeichnet, dass** Kakaobutter in einer Menge von 2 bis 4 Gewichts-%, bezogen auf das Gesamtgewicht der Zubereitung eingesetzt wird.

14. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; Thiamidol; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A, enthält.

15. Kosmetische Zubereitung nach einem der vorhergehenden Ansprüche, **dadurch gekennzeichnet, dass** die Zubereitung 2,6-Di-*tert*-butyl-*p*-kresol (INCI: BHT) enthält.

16. Kosmetische Zubereitung nach einem der vorhergehenden Ansprüche, **dadurch gekennzeichnet, dass** die Zubereitung frei ist von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen, Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern.

## Claims

1. Anhydrous cosmetic preparation comprising
a) Diisostearoyl Polyglyceryl-3 Dimer Dilinoleate
b) Polyglyceryl-4 Diisostearate
c) fermented olive oil
d) one or more oils having a surface tension of less than 31 mN/m.

2. Cosmetic preparation according to Claim 1, **characterized in that** the preparation comprises less than 1% by weight, based on the total weight of the preparation, of water.

3. Cosmetic preparation according to either of the preceding claims, **characterized in that** the preparation comprises one or more fatty alcohols.

4. Cosmetic preparation according to any of the preceding claims, **characterized in that** the preparation comprises cocoa butter.

5. Cosmetic preparation according to any of the preceding claims, **characterized in that** oils having a surface tension of less than 31 mN/m used are one or more compounds selected from the following group: octyldodecanol, caprylic/capric triglyceride (INCI: Caprylic/Capric Triglyceride), isopropyl palmitate (INCI: Isopropyl Palmitate), shea butter ethyl esters (INCI: Shea Butter Ethyl Esters), rapeseed oil (INCI: Brassica Campestris Seed Oil).

6. Cosmetic preparation according to any of the preceding claims, **characterized in that** the olive oil has been fermented by Pseudozyma sp. SY-16 [KCTC 8950P].

7. Cosmetic preparation according to any of the preceding claims, **characterized in that** the fermented olive oil used is the substance having the INCI name Pseudozyma Epicola/Olive Fruit Oil.

8. Cosmetic preparation according to any of the preceding claims, **characterized in that** Diisostearoyl Polyglyceryl-3 Dimer Dilinoleate is used in a concentration of 2% to 4% by weight, based on the total weight of the preparation.

9. Cosmetic preparation according to any of the preceding claims, **characterized in that** Polyglyceryl-4 Diisostearate is used in a concentration of 2% to 4% by weight, based on the total weight of the preparation.

10. Cosmetic preparation according to any of the preceding claims, **characterized in that** fermented olive oil is used in a concentration of 0.5% to 2% by weight, based on the total weight of the preparation.

11. Cosmetic preparation according to any of the preceding claims, **characterized in that** the total amount of oils having a surface tension of less than 31 mN/m is at least 60% by weight, based on the total weight of the preparation.

12. Cosmetic preparation according to any of the preceding claims, **characterized in that** the total amount of fatty alcohols is 2% to 4% by weight, based on the total weight of the preparation.

13. Cosmetic preparation according to any of the preceding claims, **characterized in that** cocoa butter is used in an amount of 2% to 4% by weight, based on the total weight of the preparation.

14. Cosmetic preparation according to any of the preceding claims, **characterized in that** the preparation comprises one or more active ingredients selected from the following group of compounds: alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, taurine, β-alanine, panthenol, magnolol, honokiol, tocopheryl acetate, dihydroxyacetone; thiamidol; 8-hexadecene-1,16-dicarboxylic acid, glycerylglucose, (2-hydroxyethyl)urea, vitamin E or derivatives thereof, hyaluronic acid and/or salts thereof, and/or licochalcone A.

15. Cosmetic preparation according to any of the preceding claims, **characterized in that** the preparation comprises 2,6-di-*tert*-butyl-*p*-cresol (INCI: BHT).

16. Cosmetic preparation according to any of the preceding claims, **characterized in that** the preparation is free of 3-(4-methylbenzylidene)camphor, 2-hydroxy-4-methoxybenzophenone (INCI: Oxybenzone), 2-ethylhexyl 4-methoxycinnamate (INCI: Octyl Methoxycinnamate), ethylhexyl 2-cyano-3,3-diphenylacrylate (INCI: Octocrylene), parabens (particularly methyl, propyl and butyl paraben), methylisothiazolinone, chloromethylisothiazolinone and DMDM hydantoin, polyethylene glycol ethers or polyethylene glycol esters.

## Revendications

1. Préparation cosmétique anhydre contenant
a) du dilinoléate de diisostéaroyl-polyglycéryle-3 dimère,
b) du diisostéarate de polyglycéryle-4,
c) de l'huile d'olive fermentée,
d) une ou plusieurs huiles présentant une tension de surface inférieure à 31 mN/m.

2. Préparation cosmétique selon la revendication 1, **caractérisée en ce que** la préparation contient moins de 1% en poids, par rapport au poids total de la préparation, d'eau.

3. Préparation cosmétique selon l'une des revendications précédentes, **caractérisée en ce que** la préparation contient un ou plusieurs alcools gras.

4. Préparation cosmétique selon l'une des revendications précédentes, **caractérisée en ce que** la préparation contient du beurre de cacao.

5. Préparation cosmétique selon l'une des revendications précédentes, **caractérisée en ce qu'**on utilise, comme huiles présentant une tension de surface inférieure à 31 mN/m, un ou plusieurs composés choisis dans le groupe octyldodécanol, triglycéride caprylique/caprique (INCI : Caprylic/Capric Triglyceride), palmitate d'isopropyle (INCI : Isopropyl Palmitate), ester éthylique de beurre de karité (INCI : Shea Butter Ethyl Esters), huile de colza (INCI : Brassica Campestris Seed Oil).

6. Préparation cosmétique selon l'une des revendications précédentes, **caractérisée en ce que** l'huile d'olive a été fermentée par Pseudozyma sp. SY-16[KCTC 8950P].

7. Préparation cosmétique selon l'une des revendications précédentes, **caractérisée en ce qu'**on utilise, comme huile d'olive fermentée, la substance désignée selon INCI par Pseudozyma Epicola/Olive Fruit Oil.

8. Préparation cosmétique selon l'une des revendications précédentes, **caractérisée en ce que** le dilinoléate de diisostéaroyl-polyglycéryle-3 dimère est utilisé en une concentration de 2 à 4% en poids par rapport au poids total de la préparation.

9. Préparation cosmétique selon l'une des revendications précédentes, **caractérisée en ce que** le diisostéarate de polyglycéryle-4 est utilisé en une concentration de 2 à 4% en poids par rapport au poids total de la préparation.

10. Préparation cosmétique selon l'une des revendications précédentes, **caractérisée en ce que** l'huile d'olive fermentée est utilisée en une concentration de 0,5 à 2% en poids par rapport au poids total de la préparation.

11. Préparation cosmétique selon l'une des revendications précédentes, **caractérisée en ce que** la quantité totale d'huiles présentant une tension de surface inférieure à 31 mN/m représente au moins 60% en poids par rapport au poids total de la préparation.

12. Préparation cosmétique selon l'une des revendications précédentes, **caractérisée en ce que** la quantité totale d'alcools gras représente 2 à 4% en poids par rapport au poids total de la préparation.

13. Préparation cosmétique selon l'une des revendications précédentes, **caractérisée en ce que** le beurre de cacao est utilisé en une quantité de 2 à 4% en poids par rapport au poids total de la préparation.

14. Préparation cosmétique selon l'une des revendications précédentes, **caractérisée en ce que** la préparation contient un ou plusieurs principes actifs choisis dans le groupe des composés acide alpha-liponique, acide folique, phytoène, D-biotine, coenzyme Q10, alpha-glucosylrutine, carnitine, carnosine, isoflavonoïdes naturels et/ou synthétiques, flavonoïdes, créatine, créatinine, taurine, β-alanine, panthénol, magnolol, honokiol, acétate de tocophéryle, dihydroxyacétone ; thiamidol ; acide 8-hexadécène-1,16-dicarboxylique, glycérylglucose, (2-hydroxyéthyl)urée, vitamine E ou, selon le cas, ses dérivés, acide hyaluronique et/ou ses sels et/ou licochalcone A.

15. Préparation cosmétique selon l'une des revendications précédentes, **caractérisée en ce que** la préparation contient du 2,6-di-*tert*-butyl-*p*-crésol (INCI : BHT).

16. Préparation cosmétique selon l'une des revendications précédentes, **caractérisée en ce que** la préparation est exempte de 3-(4-méthylbenzylidène)-camphre, de 2-hydroxy-4-méthoxybenzophénone (INCI : Oxybenzon), d'ester 2-éthylhexylique de l'acide 4-méthoxycinnamique (INCI : Octylmethoxycinnamate), de 2-cyano-3,3-diphénylacrylate d'éthylhexyle (INCI : Octocrylen), de parabènes (en particulier de méthylparabène, de propylparabène et de butylparabène), de méthylisothiazolinone, de chlorométhyl-isothiazolinone et de DMDM-hydantoïne, de polyéthylèneglycoléthers ou d'esters de polyéthylèneglycol.
